(19) Europäisches Patentamt
European Patent Office
Office européen des brevets

(11) Publication number: 0 083 683
A1

(12) EUROPEAN PATENT APPLICATION

(21) Application number: 82106213.0

(22) Date of filing: 12.07.82

(51) Int. Cl.³: C 07 D 263/04
C 07 D 265/06, A 61 K 31/42
A 61 K 31/535

(30) Priority: 26.10.81 US 314647

(43) Date of publication of application:
20.07.83 Bulletin 83/29

(84) Designated Contracting States:
AT BE CH DE FR GB IT LI LU NL SE

(71) Applicant: AMERICAN CYANAMID COMPANY
1937 West Main Street P.O. Box 60
Stamford Connecticut 06904(US)

(72) Inventor: Murdock, Keith Chadwick
15 Birch Street
Pearl River New York 10965(US)

(74) Representative: Wächtershäuser, Günter, Dr.
Tal 29
D-8000 München 2(DE)

(54) **Method of preparing 1,4-bis(substituted-amino)-5,8-dihydroxyanthraquinones and pharmaceutical compositions containing them.**

(57) This disclosure describes compositions of matter useful as inhibitors of transplanted mouse tumor growth and the method of inducing the regression and/or palliation of leukemia and related cancers in mammals therewith, the active ingredients of said compositions of matter being certain 1,4-bis(substituted-amino)-5,8-dihydroxyanthraquinones.

EP 0 083 683 A1

Croydon Printing Company Ltd.

TITLE MODIFIED
see front page

# METHOD OF TREATING TUMORS IN WARM-BLOODED ANIMALS WITH NOVEL COMPOSITIONS OF MATTER

## BRIEF SUMMARY OF THE INVENTION

This invention relates to novel compositions of matter useful for ameliorating cancer diseases in mammals. More particularly, it relates to therapeutic compositions containing certain 1,4-bis(substituted-amino)-5,8-dihydroxyanthraquinones which inhibit the growth of transplanted mouse tumors. The invention includes the new compositions of matter and the method of inducing the regression and/or palliation of leukemia and related cancers in mammals therewith. The 1,4-bis(substituted-amino)-5,8-dihydroxyanthraquinones of the present invention may be represented by the following structural formula:

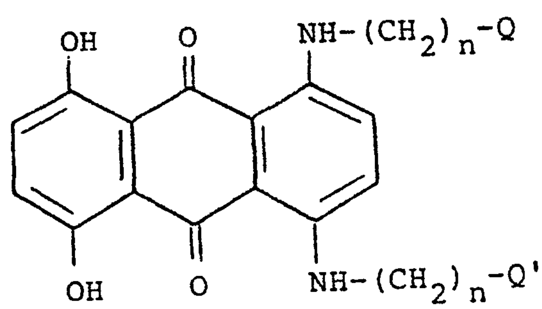

(I)

wherein n is the integer 2 or 3 and Q and Q' are each individually selected from the group consisting of moieties

of the formulae:

$-NH-(CH_2)_m-OH$ and 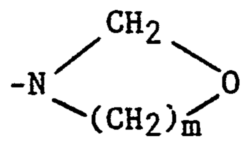

wherein m is the integer 2 or 3, with the proviso that at least one of the groups Q and Q' must be cyclic. Also included within the purview of the present invention are the leuco bases and tautomers thereof which may be represented by the following general formulae:

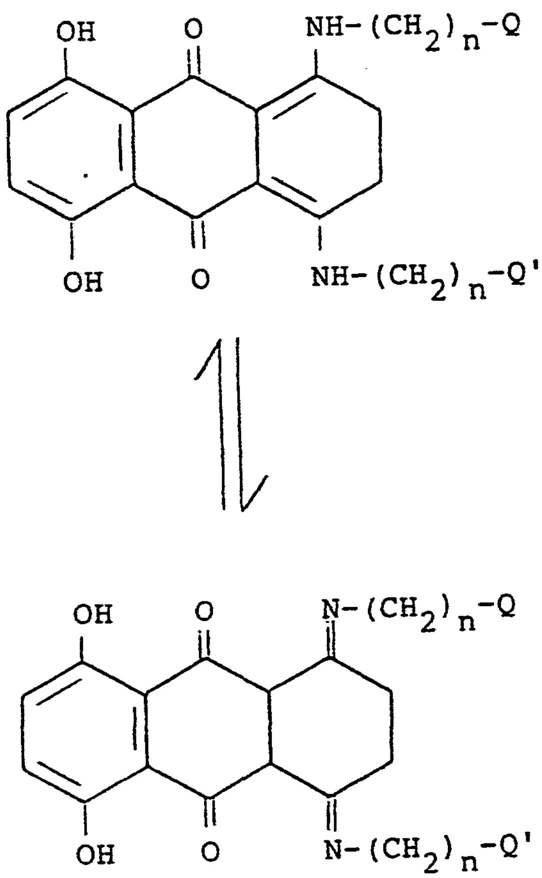

(II, LEUCO BASES)

(III, TAUTOMERIC FORM)

wherein n, Q and Q' are as hereinabove defined.

DETAILED DESCRIPTION OF THE INVENTION

Certain of the active compounds of the present invention where Q and Q' are the same and cyclic are obtainable as blue-black crystalline materials having characteristic melting points and absorption spectra and which may be purified by recrystallization or by leaching with lower alkanols.

The organic bases of this invention, (I, II and III) form non-toxic acid-addition salts with a variety of pharmacologically acceptable organic and inorganic salt--forming reagents. Thus, acid-addition salts, formed by admixture of the organic free base with 1,2 or up to eight equivalents of an acid, suitably in a neutral solvent, are formed with such acids as sulfuric, phosphoric, hydrochloric, hydrobromic, sulfamic, citric, lactic, malic, succinic, tartaric, acetic, benzoic, gluconic, ascorbic, and the like. For purposes of this invention the free bases are equivalent to their non-toxic acid-addition salts. The acid-addition salts of the organic bases of the present invention are, in general, crystalline solids, relatively soluble in water, methanol and ethanol but relatively insoluble in non-polar organic solvents such as diethyl ether, benezene, toluene, and the like.

The active compounds of the present invention may be readily prepared in accordance with the following reaction scheme:

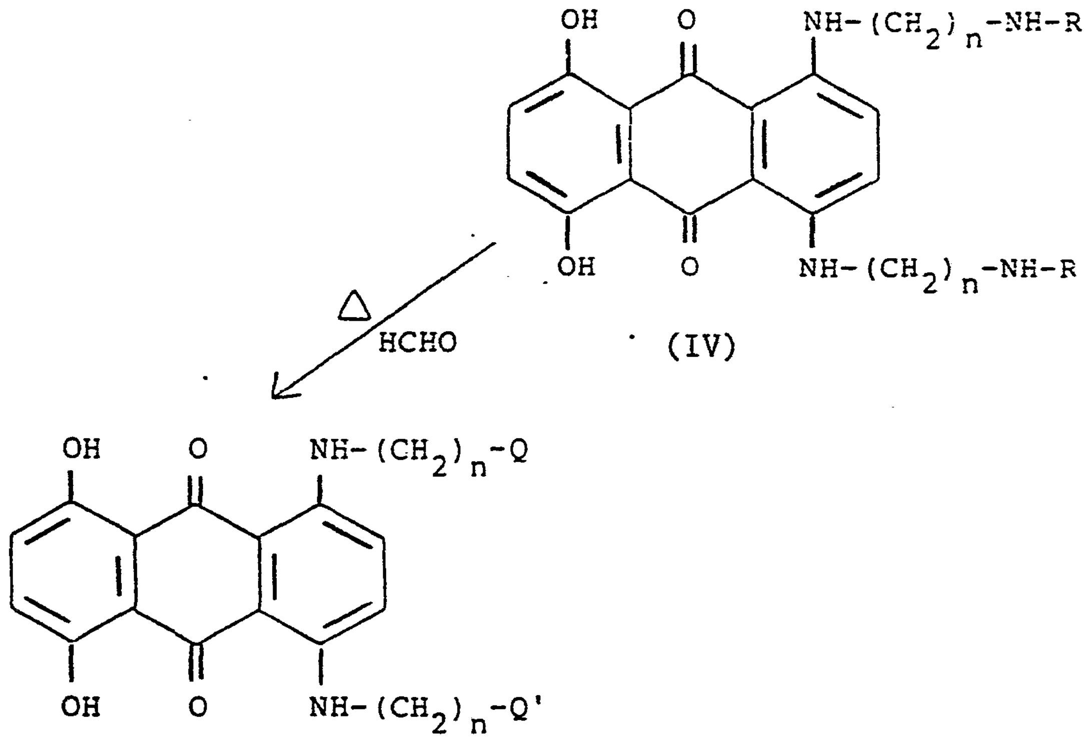


(IV)

(I)

wherein R is $-(CH_2)_m-OH$ and n, m, Q and Q' are hereinbefore defined.

In accordance with the above reaction scheme, a 1,4-dihydroxy-5,8-bis[(hydroxyalkylamino)alkylamino]anthraquinone free base (IV) may be converted to the fully cyclized product (I) by stirring and refluxing a mixture of the free base in a solvent such as benzene with two or more equivalents of aqueous formaldehyde (37.1%) for a period of 3 hours, then collecting the product by filtration and washing with benzene. Alternatively, when the free base (IV) in benzene is refluxed as described above with only one equivalent of aqueous formaldehyde, the product (I) is obtained where only one of Q and Q' is cyclic. The precursors (IV) and general reaction conditions are also set forth in U.S. Patent No. 4,197,249 which is hereby incorporated by reference.

The active compounds described herein are useful as chelating, complexing or sequestering agents. The complexes formed with polyvalent metal ions are particularly stable and usually soluble in various organic solvents. These properties, of course, render them useful for a variety of purposes wherein metal ion contamination presents a problem; e.g., as stabilizers in various organic systems such as saturated and unsaturated lubricating oils and hydrocarbons, fatty acids and waxes, wherein transition metal ion contamination accelerates oxidative deterioration and color formation. They are further useful in analyses of polyvalent metal ions which may be complexed or extracted by these materials and as metal carriers. Other uses common to sequestering agents are also apparent for these compounds.

The active compounds of the present invention also possess the property of inhibiting the growth of transplanted mouse tumors as established by the following tests.

Lymphocytic leukemia P388 test

The animals used are DBA/2 mice all of one sex, weighing a minimum of 17 g. and all within a 3 g. weight range. There are 5 or 6 animals per test group. The tumor transplant is by intraperitoneal injection of 0.5 ml. of dilute ascitic fluid containing $10^6$ cells of lymphocytic leukemia P388. The test compounds are administered intraperitoneally on days one, 5 and 9 (relative to tumor inoculation) at various doses. The animals are weighed and survivors are recorded on a regular basis for 30 days. The median survival time and the ratio of survival time for treated (T)/control (C) animals are calculated. The positive control compound is 5-fluorouracil given as a 60 mg./kg. injection. The results of this test with representative compounds of the present invention appear in Table I. The criterion for efficacy is T/C x $100 \geq 125\%$.

TABLE I

Lymphocytic Leukemia P388 Test

| Compound | Dose (mg./kg.) | Median Survival Time (Days) | T/C x 100 (Percent) | "Cures"* |
|---|---|---|---|---|
| 1,4-Dihydroxy-5,8-bis[[2-(3-oxazolidinyl)ethyl]-amino]anthraquinone (1st test) | 6.25 | >30.0 | >273 | 2/6 |
| | 3.12 | 29.5 | 268 | 1/6 |
| | 1.56 | 23.5 | 214 | |
| | 0.78 | 23.0 | 209 | |
| Control | - | 11.0 | - | |
| 5-Fluorouracil | 60 | 21.0 | 191 | |
| 1,4-Dihydroxy-5,8-bis[[2-(3-oxazolidinyl)ethyl]-amino]anthraquinone (2nd test) | 6.25 | 14.0 | 127 | |
| | 3.12 | >30.0 | >273 | |
| | 1.56 | 30.0 | 273 | 1/6 |
| | 0.78 | 22.0 | 200 | |
| | 0.39 | 23.5 | 214 | |
| | 0.19 | 23.5 | 214 | |
| | 0.10 | 19.5 | 177 | |
| Control | - | 11.0 | - | |
| 5-Fluorouracil | 60 | 21.5 | 195 | |

*"Cures" = number of survivors/total at 60 days.

Melanotic Melanoma B16

The animals used are $BDF_1$ mice, all of the same sex, weighing a minimum of 17 g. and all within a 3 g. weight range. There are normally 10 animals per test group. A one-gram portion of melanotic melanoma B16 tumor is homogenized in 10 ml. of cold balanced salt solution and a 0.5 ml. aliquot of the homogenate is implanted intraperitoneally into each of the test mice. The test compounds are administered intraperitoneally on days one through 9 (relative to tumor inoculation) at various doses. The animals are weighed and survivors are recorded on a regular basis for 60 days. The median survival time and the ratio of survival time for treated (T)/control (C) animals are calculated. The positive control compound is 5-fluorouracil given as a 20 mg./kg. injection. The results of this test with representative compounds of the present invention appear in Table II. The criterion for efficacy is T/C x 100 $\geq$125%.

TABLE II

Melanotic Melanoma B16 Test

| Compound | Dose (mg./kg.) | Median Survival Time (Days) | T/C x 100 (Percent) |
|---|---|---|---|
| 1,4-Dihydroxy-5,8-bis[[2--(3-oxazolidinyl)ethyl]-amino]anthraquinone | 1.5 | 55.5 | 292 |
| | 0.75 | 46.0 | 242 |
| | 0.37 | 49.5 | 260 |
| | 0.19 | 31.0 | 163 |
| | 0.09 | 29.0 | 153 |
| Control | - | 19.0 | |
| 5-Fluorouracil | 20.0 | 26.0 | 137 |

Also embraced within the purview of the present invention are therapeutic compositions of matter useful for ameliorating cancer diseases in mammals and containing the 1,4-bis(substituted-amino)-5,8-dihydroxyanthraquinones (I) of the present invention. This aspect of the invention includes the novel compositions of matter and the method of inducing the regression and/or palliation of leukemia and related cancers in mammals therewith.

The active ingredients of the therapeutic compositions of the present invention inhibit transplanted mouse tumor growth and induce regression and/or palliation of leukemia and related cancers in mammals when administered in amounts ranging from about 0.3 mg. to 100 mg. per square meter of body surface area per day. The interrelationship of dosages for animals of various sizes and species and humans (based on mg./$m^2$ of surface area) is described by Freireich, E. J., et al., Quantitiative Comparison of Toxicity of Anticancer Agents in Mouse, Rat, Hamster, Dog, Monkey and Man. Cancer Chemother. Rep., 50, No. 4, 219-244, May 1966. A preferred dosage regimen for optimum results would be from about 1.0 mg./$m^2$/day to about 30 mg./$m^2$/day and such dosage units are employed that a total of from about 0.5 mg. to about 60 mg. of the active compound for a subject of about 70 kg. of body weight are administered in a 24 hour period. This dosage regimen may be adjusted to provide the optimum therapeutic response. For example, several divided doses may be administered daily or the dose may be proportionally reduced as indicated by the exigencies of the therapeutic situation. The active compounds may be administered by the intravenous, intramuscular, or subcutaneous routes.

The active compounds may be administered parenterally or intraperitoneally. Solutions or dispersions of the active compound as a free base can be prepared in water suitably mixed with a surfactant such as hydroxypropylcellulose. Dispersions can also be prepared in glycerol, liquid polyethylene glycols, and mixtures thereof

and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases it will be preferable to include isotonic agents, for example sugars or sodium chloride. Prolonged absorption of the injectable compositions can be brought about by the use in the compositions of agents delaying absorption, for example aluminum monostearate and gelatin.

Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the

preparation of sterile injectable solutions, the preferred methods of preparation are vacuum drying and the freeze--drying technique which yield a powder of the active ingredient plus any additional desired ingredient from a previously sterile-filtered solution thereof.

As used herein, "pharmaceutically acceptable carrier" includes any and all solvents, dispersion media, coatings, antibacterial and antifungal agents, isotonic and absorption delaying agents and the like. The use of such media and agents for pharmaceutical active substances is well known in the art. Except insofar as any conventional media or agents is incompatable with the active ingredient, its use in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitations inherent in the art of compounding such an active material for the treatment of disease in living subjects having a diseased condition in which bodily health is impaired as herein disclosed in detail.

The principal active ingredient is compounded for convenient and effective administration in effective amounts with a suitable pharmaceutically-acceptable carrier in dosage unit form as hereinabefore disclosed.

A unit dosage form can, for example, contain the principal active compound in amounts ranging from about 0.1 to about 200 mg., with from about one to about 30 mg. being preferred. Expressed in proportions, the active compound is generally present in from about 0.1 to about 100 mg./ml. of carrier. In the case of compositions containing supplementary active ingredients, the dosages are determined by reference to the usual dose and manner of administration of the said ingredients.

Regression and palliation of cancers are attained, for example, using intraperitoneal administration. A single intravenous dosage or repeated daily dosages can be administered. Daily dosages up to about 5 or 10 days are often sufficient. It is also possible to dispense one daily dosage or one dose on alternate or less frequent days. As can be seen from the dosage regimens, the amount of principal active ingredient administered is a sufficient amount to aid regression and palliation of the leukemia or the like, in the absence of excessive deleterious side effects of a cytotoxic nature to the hosts harboring the cancer. As used herein, cancer disease means blood malignancies such as leukemia, as well as other solid and non-solid malignancies such as the melanocarcinomas, lung carcinomas, and mammary tumors. By regression and palliation is meant arresting or retarding the growth of the tumor or other manifestation of the disease compared to the course of the disease in the absence of treatment.

This invention will be described in greater detail in conjunction with the following specific examples.

## Example 1

## 1,4-Dihydroxy-5,8-bis[[2-(3-oxazolidinyl)ethyl]amino]-anthraquinone

A) A mixture of 30.0 g. of 1,4-bis[2-(2-hydroxyethylamino)ethylamino]-5,8-dihydroxyanthraquinone dihydrochloride (prepared as described in Example 24 of U.S. Patent 4,197,249) and 300 ml. of methanol is chilled in an ice bath in a Dewar flask. The mixture is saturated with ammonia gas and is allowed to stand at 0°C. for one hour with the continuous slow addition of ammonia gas and with periodic stirring. The solid is collected by filtration and washed by slurrying with five 150 ml. portions of methanol saturated with ammonia gas to give 22.9 g. of 1,4-bis[[2-(2-hydroxyethylamino)ethyl]amino]-5,8-dihydroxyanthraquinone as blue-black micro rods, m.p. 175--178°C.

B) A suspension of 3.11 g. of the preceding compound in 55 ml. of benzene and 3.14 ml. of (37.1%) aqueous formaldehyde is stirred and heated under reflux using a Dean-Stark trap. About 2.2 ml. of water is collected as distillate over a 3 hour reflux period. The reaction suspension is allowed to cool and the solid is collected and washed with benzene. The dried solid is dissolved in 90 ml. of hot chloroform and the solution is filtered. The filtrate is cooled, then 180 ml. of ether is added to precipitate a bulky solid. The solid is collected and washed with ether to give 2.39 g. of the product of the Example as blue-black crystals, m.p. 203-204°C.

Example 2

1,4-Dihydroxy-5-[[2-(2-hydroxyethylamino)ethyl]amino]-8-[[2-(3-oxazolidinyl)ethyl]amino]anthraquinone

A suspension of 3.11 g. of 1,4-bis[[2-(2-hydroxyethylamino)ethyl]amino]-5,8-dihydroxyanthraquinone, (prepared as described in Example 1, part A) in 55.0 ml. of benzene and 1.05 ml. of aqueous formaldehyde (37.1%) is refluxed for 3 hours as described in Example 1, part B. The suspension is cooled and the precipitate is collected by filtration and washed with benzene to give the desired product.

Example 3

1,4-Bis[[2-(dihydro-2H-1,3-oxazin-3(4H)-yl)ethyl]-amino]-5,8-dihydroxyanthraquinone

A) A mixture of 30.0 g. of 1,4-bis[[2-[(3-hydroxypropyl)amino]ethyl]amino]-5,8-dihydroxyanthraquinone dihydrochloride (prepared as described in Example 30 of U.S. Patent 4,197,249) and 300 ml. of methanol is saturated with ammonia gas by the procedure of Example 1, part A, to yield the corresponding free base compound, 1,4-bis[[2-[(3-hydroxypropyl)amino]-ethyl]amino]-5,8-dihydroxyanthraquinone.

B) A suspension of 3.32 g. of the preceding free base in 55.0 ml. of benzene and 3.14 ml. of (37.1%) aqueous formaldehyde is treated as described in Example 1, part B, to yield the product of the Example.

Example 4

1-[[2-(Dihydro-2H-1,3-oxazin-3(4H)-yl)ethyl]-amino]-5,8-dihydroxy-4-[[2-[(3-hydroxypropyl)amino]ethyl]amino]-anthraquinone

A suspension of 3.32 g. of 1,4-bis[[2-[(3-hydroxypropyl)amino]ethyl]amino]-5,8-dihydroxyanthraquinone (prepared as described in Example 3, part A) in 55.0 ml. of benzene and 1.05 ml. of (37.1%) aqueous formaldehyde is refluxed and treated as described in Example 2 to give the product of the Example.

Example 5

Preparation of Parenteral Suspension

In a solution of 700 ml. of propylene glycol and 200 ml. of water for injection is suspended 2.0 g. of 1,4-dihydroxy-5,8-bis[[2-(3-oxazolidinyl)ethyl]amino]anthraquinone with stirring. After the suspension is complete the volume is made up to 1000 ml. with water for injection. The formulation is sterilized, filled into 5.0 ml. ampoules each containing 2.0 ml. (representing 4 mg. of drug) and sealed under nitrogen.

Example 6

Parenteral Suspension

The active compound in powder form is sterilized by ethylene oxide sterilization. The sterilized powder is aseptically filled into vials in dosage unit form and the vials are sealed. Immediately prior to use the powder is suspended by the addition of a suitable sterile diluent. The resulting suspension may be sonicated if necessary to promote dispersion. (This mode of suspension is advantageous for compounds which might undergo some hydrolysis on long standing in an aqueous medium.)

,638

## CLAIMS

We claim:

1. The method of inducing regression of leukemia and/or inhibiting growth of tumors in a mammal comprising administering to said mammal an effective amount of a compound selected from the group consisting of those of the formula:

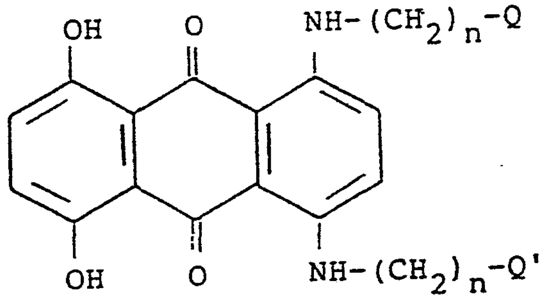

wherein n is the integer 2 or 3 and Q and Q' are each individually selected from the group consisting of moieties of the formulae:

$-NH-(CH_2)_m-OH$ and 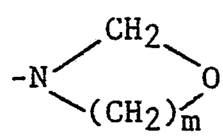

wherein m is the integer 2 or 3 with the proviso that at least one of Q and Q' must be cyclic; the leuco bases and tautomers thereof; and the pharmacologically acceptable acid-addition salts thereof.

2. The method according to Claim 1 wherein the compound is 1,4-dihydroxy-5,8-bis[[2-(3-oxazolidinyl)-ethyl]amino]anthraquinone.

3. The method according to Claim 1 wherein the compound is 1,4-dihydroxy-5-[[2-(2-hydroxyethylamino)-ethyl]amino]-8-[[2-(3-oxazolidinyl)ethyl]amino]anthraquinone.

4. The method according to Claim 1 wherein the compound is 1,4-bis[[2-(dihydro-2H-1,3-oxazin-3(4H)-yl)-ethyl]amino]-5,8-dihydroxyanthraquinone.

5. The method according to Claim 1 wherein the compound is 1-[[2-(dihydro-2H-1,3-oxazin-3(4H)-yl)ethyl]-amino]-5,8-dihydroxy-4-[[2-[(3-hydroxypropyl)amino]ethyl]-amino]anthraquinone.

6. A pharmaceutical composition in dosage unit form comprising from about 0.5 mg. to about 60.0 mg. of a compound selected from the group consisting of those of the formula:

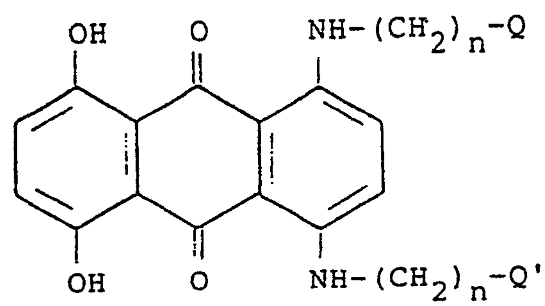

wherein n is the integer 2 or 3 and Q and Q' are each individually selected from the group consisting of moieties of the formulae:

$-NH-(CH_2)_m-OH$ and 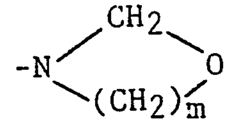

wherein n is the integer 2 or 3 with the proviso that at least one of Q and Q' must be cyclic; the leuco bases and tautomers thereof; and the pharmacologically acceptable acid-addition salts thereof; in association with a pharmaceutical carrier.

7. A composition according to Claim 6 wherein the compound is 1,4-dihydroxy-5,8-bis[[2-(3-oxazolidinyl)-ethyl]amino]anthraquinone.

8. A composition according to Claim 6 wherein the compound is 1,4-dihydroxy-5-[[2-(2-hydroxyethylamino)-ethyl]amino]-8-[[2-(3-oxazolidinyl)ethyl]amino]anthraquinone.

9. A composition according to Claim 6 wherein the compound is 1,4-bis[[2-(dihydro-2H-1,3-oxazin-3(4H)--yl)ethyl]amino]-5,8-dihydroxyanthraquinone.

10. A composition according to Claim 6 wherein the compound is 1-[[2-(dihydro-2H-1,3-oxazin-3(4H)-yl)-ethyl]amino-5,8-dihydroxy-4-[[2-[(3-hydroxypropyl)amino]-ethyl]amino]anthraquinone.

11. The process of preparing compounds of the formula:

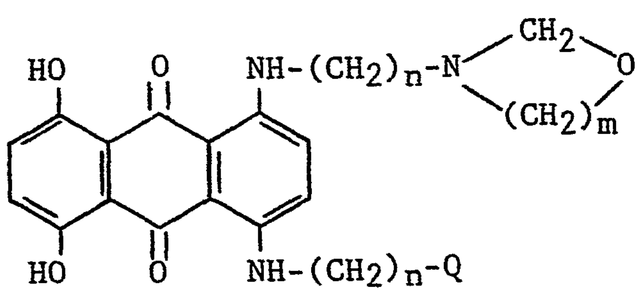

wherein n is 2 or 3, m is 2 or 3, and Q is a moiety of the formulae:

$-NH-(CH_2)_m-OH$ and 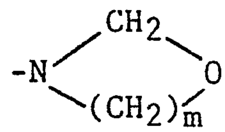

wherein m is as hereinabove defined which comprises treating a compound of the formula:

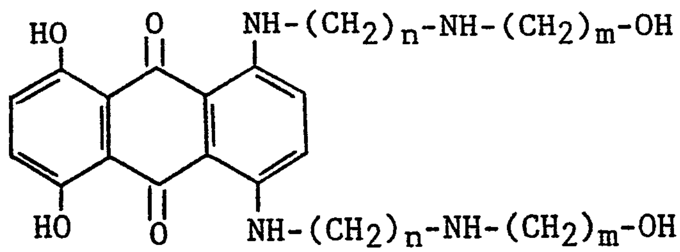

with one or two molar equivalents of formaldehyde in benzene at the reflux temperature thereof for a period of time of about three hours.

0083683

European Patent Office

# PARTIAL EUROPEAN SEARCH REPORT

which under Rule 45 of the European Patent Convention shall be considered, for the purposes of subsequent proceedings, as the European search report

Application number: EP 82 10 6213.0

## DOCUMENTS CONSIDERED TO BE RELEVANT

| Category | Citation of document with indication, where appropriate, of relevant passages | Relevant to claim |
|---|---|---|
| | EP - A2 - 0 037 486 (AMERICAN CYANAMID ) | |
| X | * example 44 * | 11 |
| Y | * examples 44, 45 and page 12, line 22, until page 13, line 28 * | 6 |
| | -- | |
| Y | DE - A1 - 2 835 661 (AMERICAN CYANAMID) * claim 1, examples 44, 45 and page 57, last paragraph * | 6 |
| | -- | |
| A | EP - A1 - 0 021 622 (AMERICAN CYANAMID) * claim 12 * | 6 |
| | -- | |
| P,A | EP - A1 -0 042 940 (AMERICAN CYANAMID) * abstract * | 6 |
| | ---- | |

CLASSIFICATION OF THE APPLICATION (Int. Cl.³)

C 07 D 263/04
C 07 D 265/06
A 61 K 31/42
A 61 K 31/535

TECHNICAL FIELDS SEARCHED (Int. Cl.³)

A 61 K 31/00
C 07 D 263/04
C 07 D 265/06

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 6-11
Claims searched incompletely: --
Claims not searched: 1-5
Reason for the limitation of the search:

See Article 52 (4) of the European Patent Convention. Method for treatment of the human or animal body by therapy.

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

| Place of search | Date of completion of the search | Examiner |
|---|---|---|
| Berlin | 18-01-1983 | PHILLIPS |